# EUROPEAN PATENT APPLICATION

(11) **EP 1 343 006 A1**
(43) Date of publication of application: **10.09.2003**
(21) Application number: 01982776.5
(22) Date of filing: 14.11.2001
(51) Int. Cl.: G01N 33/15, G01N 33/50

(54) **METHOD OF PROFILING PROTEIN**

(30) Priority: 15.11.2000 JP 2000348054; 27.12.2000 JP 2000396950
(71) Applicant: ITAI, Akiko, Tokyo 113-0033 (JP)
(72) Inventor: ITAI, Akiko, Tokyo 113-0033 (JP)
(74) Representative: Sternagel, Fleischer, Godemeyer & Partner Patentanwälte
(86) International application number: JP0109940
(87) International publication number: WO02040990

(57) **Abstract**

A method of predicting biological functions and/or diseases to which an arbitrary target protein is related by applying a probe means constructed on the basis of the information of the target protein to a biomaterial or an animal and by analyzing the effects thereof on the behaviors of one or more monitoring molecules having known and/or unknown biological functions. For example, by analyzing a direct or indirect relation of the target protein to a molecule included in known biological molecule network information, the biological functions and/or diseases to which the target protein is related can be predicted.

## Description

### Technical Field

The present invention relates to a method of predicting protein functions, more specifically, it relates to a method of predicting biological functions and/or diseases in which proteins are involved and confirming validity as target for drug discovery.

### Background Art

Recently, existence of many proteins has been revealed from studies of genomes and genes. However, there are many proteins that are not utilized because their biological functions are unknown even though the amino acid sequences are known. There is an empirical rule wherein biochemical functions are analogous among proteins in which amino acid sequences have certain homology. Prediction of functions or classification into a family are routinely carried out for an arbitrary protein on the basis of sequence information. For example, concerning a protein kinase, a fact that a target protein has a biochemical function of phosphorylation of a protein and a fact that a protein belongs to a protein kinase family can be predicted to some extent by examining homology to a sequence of a protein with known functions or existence of a specific partial sequence (a motif) by using a computer. However, among protein kinases, they have different biological functions and physiological roles depending on a substrate protein to be phosphorylated, a protein acting as an effector, an organ or an intracellular site where they function, period of time of expression and the like. It is known that there exist more than 2,000 kinds of protein kinases in human body.

Therefore, it is necessary to elucidate biological functions of a protein for use as a target for drug discovery in process of new drug development. However, an effective method to elucidate functions has not been established so far. Experimental elucidation is ultimately required; however, elucidation of functions by a vast amount of experiments, one and all by trial and error, is apparently difficult even for a single protein from viewpoints of labor, time, and cost. Furthermore, generally, information on three-dimensional structure of a protein may be useful for prediction of biochemical functions, but only slightly helpful for elucidation of biological functions.

As a method of predicting biological functions of a protein only with information on an amino acid sequence, there is a method of generating an animal wherein a gene coding a target protein is knocked-out or over-expressed. However, when the protein is most essential, an offspring may sometimes not be born, or even when an offspring is born, differences from a normal animal are often not clear, and therefore, a possibility of successful prediction or elucidation of biological functions of the protein is not so high. Furthermore, it is possible to examine an influence on a protein expression by preparing an antisense molecule to mRNA corresponding to the protein. However, interpretation of experimental results may often be difficult, and therefore, the method has not become an effective means of predicting biological functions and roles in an organism of the protein.

Recently, more attention has been paid for searching a protein that can possibly be a candidate for a target of novel drug discovery, by using techniques for analyses of disease-associated genes and their protein expression and the like. For this reason, it becomes necessary to judge in an early stage whether or not a protein is adequate as a target for new drug discovery. Even if a function of a protein is predictable to some extent, when the protein is a novel target for drug discovery, it is necessary to establish a method of judging whether or not it is appropriate to develop a drug by using the protein as a target, such that the drug is effective for target diseases with less side effects from a mode of action, before beginning intensive researches and developments of the drug.

### Disclosure of Invention

An object of the present invention is to provide a method of profiling an arbitrary protein regarding its relation to biological functions and diseases. To be more specific, the object is to provide a prediction method based on a typical simple experiment to elucidate biological functions. Another object of the present invention is to provide, for an arbitrary protein with unknown functions where only sequence information is available, a method which enables simple prediction as to what biological function is related or what disease is related to said protein. Further object of the present invention is to provide a method of obtaining information, by carrying out the aforementioned prediction before the beginning of a full-scale development of a drug, whether or not the target protein is appropriate as a target for drug discovery and what kind of risk of a side effect is expected when the target protein is used as a target for drug discovery.

As a result of zealous endeavor to achieve the foregoing objects, the inventors found that the aforementioned object can be achieved by measuring, analyzing, and comparing behaviors of a group of molecules (monitoring molecules) other than the target protein, and corresponding the results with a known biomolecule network information, wherein the behaviors are induced when a probe means to the target protein group (a ligand, an antisense, an administration of monoclonal antibody, or a knockout or a transgenic operation of a corresponding gene) is applied to a biomaterial or an animal.

More specifically, the aforementioned objects can be achieved by employing the following procedures. First, a probe means to the target protein is obtained, for example, by any one of the following methods.
(1) Based on sequence information on a gene of the target protein, an antisense oligonucleotide to mRNA that codes the protein is designed and synthesized. (2) A specific ligand to the protein is obtained by means such as a virtual screening using computer based on the crystal structure information when it is available, and when it is not available, based on a three-dimensional structure of the target protein constructed by a homology modeling using structure information of an analogous protein. When samples of the target protein, an appropriate compound library, and a high throughput-screening device are available, it is possible to obtain a specific ligand by an experimental random screening. (3) A vector is designed and obtained in which DNA is incorporated for gene destruction by insertion of a marker gene into a gene encoding the target protein with a technique such as a homologous recombination. (4) A vector is designed and obtained in which a gene encoding the protein is incorporated. (5) In addition, a means is obtained for destruction or modulation of the protein function by means such as RNA interference, a ribozyme, and a specific antibody.

A condition wherein the protein function is temporarily or permanently destructed or modulated is then brought about by applying the obtained probe means to an organism (organelle, cell, organ, tissue, animal body and the like). Then, for each of the destructed or modulated organism and the organism without those operations, behaviors (amount of expression and/or amount of presence and others) of a group of biomolecules (which are referred to as "monitoring molecules") other than the target protein, such as various proteins, mRNA, other small molecules including hormones, are measured to detect a quantitative or a temporal fluctuation caused by the destruction or the modulation of the protein function. At this time, the monitoring molecule is desirably a molecule whose biological functions are already known and included in biomolecule network information.

When the monitoring molecule is included in the biomolecule network information, it is possible to predict biological functions or diseases to which the target protein is related by analyzing the obtained experimental data in relation with meaning of each monitoring molecule in the biomolecule network information and temporal fluctuation. On the basis of this result and biochemical functions predictable by a sequence homology with a known protein and the presence or absence of a motif sequence, it will be possible to carry out an effective and appropriate experiment rather than trial and error regarding the target protein, which will be helpful for an elucidation of the biological functions. As a result, it will be possible to predict what meaning the target protein has to what disease, whether or not it is appropriate to use the protein as a target for drug discovery, or what side effects are predicted when a drug is developed by using the target protein as a target for drug discovery.

When the monitoring molecule is not included in the biomolecule network information or the biomolecule network is not utilized, obtainable information is more less than the analysis using the biomolecule network. However, it will be possible to arrange the result of modulation by the application of the aforementioned probe means in a certain order as independent data for each monitoring molecule, to express as the size of a figure or a digit or a barcode for comparison. For example, if a barcode notation, indicating a condition where a specific ligand is administered to a biomaterial of a particular disease, is more similar to a barcode notation in a normal condition rather than a barcode notation of no ligand administration, it can be recognized that the protein is more desirable as a target for drug discovery.

The following is a schematic conceptual flow of the method of the present invention.

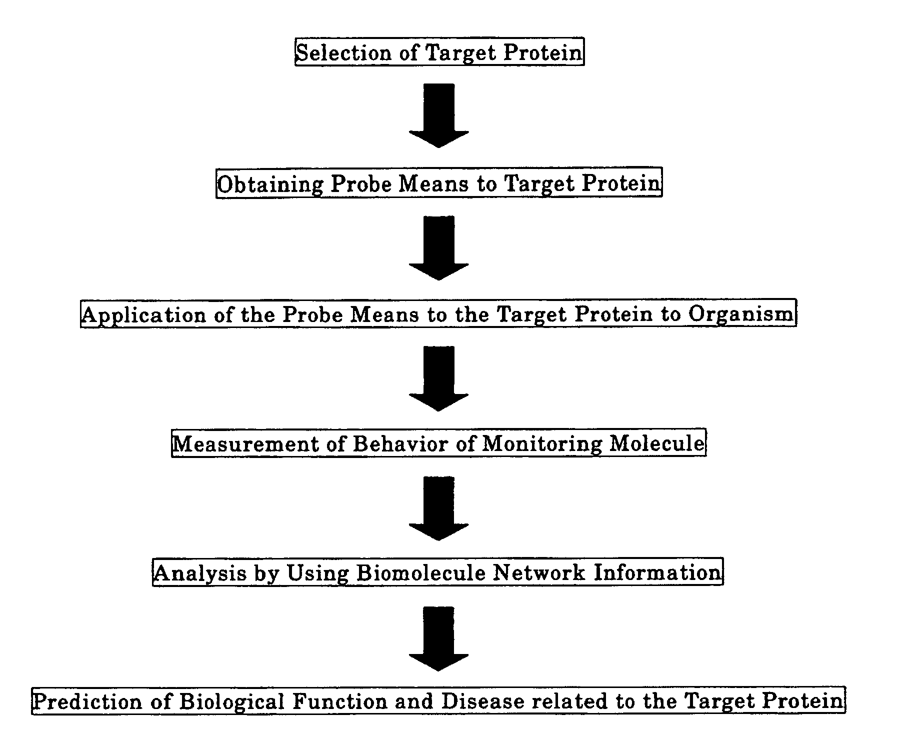

According to preferred embodiments of the invention, provided are the aforementioned method wherein a monitoring molecule is a protein with a large fluctuation of expression by applying the probe means or a protein derived from mRNA with a large fluctuation of expression by applying the probe means, and/or a group of specifically designated monitoring molecules; the aforementioned method in which the group of monitoring molecules are selected so as to have relation to variety of biological functions as much as possible; the aforementioned method in which the group of monitoring molecules are selected from a group comprising a group of molecules which is related to specific diseases and/or biological functions and a group of molecules which express and/or function in a specific organ or a specific tissue; the aforementioned method in which the biomolecule network information is a biomolecule network information indicating a linkage of an interaction in a function and/or a biosynthesis among biomolecules with known biological functions; the aforementioned method in which the biomolecule network information is a molecule function network information including information on a relation between a biomolecule and a bioevent in addition to the information indicating the linkage of the interaction in the function and/or the biosynthesis among biomolecules with known biological functions.

According to more preferred embodiments, provided are the aforementioned method wherein the specific ligand to the target protein is designed by using virtual screening; the aforementioned method in which the virtual screening includes a step of searching a compound database based on an automatic docking considering binding modes and all degrees of freedom of ligand conformations; the aforementioned method wherein the virtual screening is a search method of a compound database based on the automatic docking method ADAM; the aforementioned method which comprises a step wherein two or more specific ligands or a combination of a specific ligand and an antisense molecule to a single target protein are administered by two or more doses or concentrations, and a fluctuation of an amount of expression or an amount of presence of the monitoring molecule is measured and analyzed as a biological response.

Furthermore, according to the present invention, provided are a method of predicting a relation between the target protein and the molecules on the biomolecule network information, which comprises steps of searching other protein with an unknown biological function whose expression fluctuates remarkably by applying the probe means which is obtained based on the information on the target protein, searching one or more proteins whose expression fluctuates remarkably by applying the probe means designed for the obtained other protein, and repeating the aforementioned steps until one of the aforementioned proteins whose expression fluctuates remarkably matches with one of the molecules in the biomolecule network information; and a method of predicting a relation between two or more proteins including the target protein with unknown biological functions and the molecules in the known biomolecule network information by analyzing influence on the behavior of the monitoring molecule by applying each probe means to a biomaterial or an animal, wherein said probe means is based on each of the target protein and other protein with an unknown biological function which is known to interact directly with the target protein. In these methods, a preferred embodiment includes the method of predicting a relation with biological functions or diseases for two or more proteins with unknown biological functions including the target protein, wherein the behavior is an amount of expression and/or an amount of presence.

Moreover, according to the present invention, provided are the aforementioned method which uses a group of the monitoring molecules selected so as to include a group of molecules selected from a group consisting of a group of molecules included in the known biomolecule network information and related to a specific disease and/or a bioevent, a group of molecules expressed and/or generated in a specific organ, a group of molecules which exists and operates in a specific organ, and a group of molecules involved in bioevents as many as possible; a set of antibodies which enables to simultaneously quantify and/or detect the aforementioned group of the monitoring molecules; the aforementioned method in which a screening of a protein related to the target disease or the biological function is carried out by quantitative measurement of the monitoring molecule by using the aforementioned set of antibodies; a method of selecting a group of proteins involved in an arbitrary disease as a candidate for a target for drug discovery, a candidate for a protein drug, or a candidate for an antigen for an antibody drug on the basis of the biomolecule network information; a method of predicting a side effect when a certain protein is regarded as a candidate for a target for drug discovery, a candidate for a protein drug, or a candidate for an antigen for an antibody drug, for the purpose of developing a drug for an arbitrary disease or a symptom based on the biomolecule network information; and a method of selecting an optimum candidate from two or more candidates for a target for drug discovery, candidates for a protein drug, or candidates for an antigenic protein of an antibody drug, for the purpose of developing a drug for an arbitrary disease or a symptom on the basis of the biomolecule network information.

Examples of typical methods provided by the present invention include the following methods. However, the scope of the present invention is not limited to these examples.
1. A method of predicting biological functions and/or diseases to which the target protein is related, by applying the probe means based on an arbitrary target protein to a biomaterial or an animal, and by measuring and analyzing an influence on the behavior of one or more of the monitoring molecules with known and/or unknown biological functions.
2. A method of predicting biological functions and/or diseases to which the target protein is related, by applying the probe means based on an arbitrary target protein to a biomaterial or an animal, by measuring influence on the behavior of one or more of the monitoring molecules with known biological functions, and by analyzing a direct or an indirect relation with a molecule included in the known biomolecule network information of the target protein.
3. A method of profiling the target protein as for a role or a function of an arbitrary target protein in an organism, by measuring, describing, comparing and/or analyzing influence on a group of biomolecules other than the target protein with the probe means based on the target protein.
4. The method described in one of the aforementioned 1 through 3, which is characterized by profiling the target protein by measuring influence by the application of the probe means on the behavior of one or more of the monitoring molecules and by fingerprinting their strength expressed by a size of a figure or a number or a bar code.
5. The method described in one of the aforementioned 1 through 4, wherein the biological function of the target protein is unknown.
6. The method described in one of the aforementioned 1 through 5, wherein the target to which the probe means is applied is an animal or a biomaterial comprising an organelle, a cell, a tissue, or an organ.
7. The method described in one of the aforementioned 1 through 6, wherein the probe means is a specific ligand, an antisense molecule, a monoclonal antibody, or knockout or transgenic operation of a gene corresponding to the target protein.
8. The method described in one of the aforementioned 1 through 7, wherein the application of the probe means includes a process of administering a specific ligand, an antisense molecule, or a monoclonal antibody, and/or includes a process of generating an animal treated with knockout or transgenic operation of a gene corresponding to the target protein.
9. The method described in one of the aforementioned 1 through 8, wherein the biological response when the probe means is applied to a biomaterial or an animal is measured with a behavior of one or more of the monitoring molecules selected from the biomolecules other than the target protein.
10. The method described in one of the aforementioned 1 through 9, wherein the behavior of the monitoring molecule is quantitative or temporal fluctuation of an amount of expression and/or an amount of presence of each molecule.
11. The method described in one of the aforementioned 1 through 10, wherein the monitoring molecule is a biomolecule whose biological function is known or which is predicted to be related to a biomolecule with known biological functions, and wherein the said molecule is included in the biomolecule network information.
12. The method described in one of the aforementioned 1 through 11, wherein the monitoring molecule is a protein or mRNA whose fluctuation of the expression amount is large when the probe means is applied, and/or a specifically designated biomolecule.
13. The method described in one of the aforementioned 1 through 12, wherein the monitoring molecule is a group of molecules selected so as to be related to variety of biological functions as wide as possible.
14. The method described in one of the aforementioned 1 through 13, wherein the monitoring molecule is selected from a group consisting of a group of molecules related to specific diseases and/or biological functions and a group of molecules which express and/or function in a specific organ or a specific tissue.
15. The method described in one of the aforementioned 1 through 14, wherein the biomolecule network information is a molecule network information indicating a linkage of interactions in the functions and/or biosynthesis among the biomolecules with known biological functions.
16. The method described in one of the aforementioned 1 through 15, wherein the biomolecule network information is a molecule network information containing information of a relation between the biomolecule and the bioevent in addition to the information indicating a linkage of interactions in the function and/or biosynthesis among the biomolecules with known biological functions.
17. The method described in one of the aforementioned 2 through 16, wherein the biomolecule network information is the molecule function network information created by the method described in the specification of the Japanese Patent Application No. 2000-276699.
18. The method described in one of the aforementioned 1 through 17, which includes the steps of independently applying two or more probe means to a single target protein, and then comprehensively analyzing each influence on the fluctuation of the monitoring molecule.
19. The method described in one of the aforementioned 1 through 18, which includes the steps of administering a specific ligand, an antisense molecule, or a monoclonal antibody to a biomaterial or an animal at two or more doses or concentrations, and measuring and analyzing a quantitative or temporal fluctuation of an amount of expression or an amount of presence of the monitoring molecule.
20. A method which comprises the steps of searching a group of proteins whose expressions change considerably by applying the probe means based on the target protein, and searching a group of proteins whose expression changes considerably by applying the probe means based on one of the above, and further comprises the step of correlating between molecules on the biomolecule network and the target protein by repeating the aforementioned steps until one of the aforementioned proteins whose expression fluctuates considerably coincides with any of the molecules included in the known biomolecule network information, and predicting a relation of the target protein with a biological function or a disease based on the correlation.
21. A method which comprises the steps of applying the probe means based on each of the target protein and other protein with unknown biological functions which are known to interact directly with the target protein, to the same biomaterial or an animal, correlating two or more proteins with unknown biological functions including the target protein with molecules contained in the known biomolecule network information by an comprehensive analysis of an influence of each protein on a behavior of a monitoring molecule, and predicting a relation of two or more proteins including the target protein with biological functions or diseases based on the correlation.
22. The method described in any one of the aforementioned 1 through 21, which verifies the validity of the target protein as a target for drug discovery, the validity of the target protein as a drug, the validity of the target protein when its antibody is used as a drug, by measuring and analyzing a fluctuation of the behavior of the monitoring molecule by the application of the probe means based on the target protein.
23. The method described in any one of the aforementioned 1 through 22 wherein a protein which can be a target for drug discovery, a protein which can be a protein drug, or a protein which can be an antigen for an antibody drug is selected for the purpose of drug discovery of an arbitrary disease based on the biomolecule network information.
24. A method of selecting an appropriate candidate by applying the method described in any one of the aforementioned 1 through 23 by comparing two or more candidates for a target for drug discovery, candidates for a protein drug, or candidates of an antigenic protein for an antibody drug which are extracted based on the known biomolecule network information for an arbitrary disease.
25. The method described in any one of the aforementioned 1 through 24, which predicts a side effect when a drug is developed by using the target protein as a target for drug discovery, a side effect when the target protein itself is developed as a drug, or a side effect when an antibody to the target protein is used as a drug, by measuring and analyzing a fluctuation on a behavior of the monitoring molecule when the probe means based on a target protein is applied.
26. The method described in any one of the aforementioned 1 through 25, wherein a side effect is predicted for a protein which can be a target for drug discovery, for a protein which can be a protein drug, or for a protein which can be an antigen of an antibody drug, for the purpose of development of a drug for an arbitrary disease or a symptom based on the biomolecule network information.
27. The method described in any one of the aforementioned 1 through 26, wherein two or more specific ligands created to an arbitrary protein are employed.
28. The method described in any one of the aforementioned 1 through 27, wherein the specific ligand to the target protein is created by a virtual screening.
29. The method described in the aforementioned section 28, wherein the virtual screening is a search method of a compound database based on an automatic docking considering binding modes and all degrees of freedom of a ligand conformations.
30. The method described in the aforementioned 28, wherein the virtual screening is a search method of a compound database based on the automatic docking method ADAM.
31. A set of antibodies which enables to simultaneously quantify and/or detect the group of the monitoring molecules described in any one of the aforementioned 1 through 27.
32. The method described in any one of the aforementioned 1 through 27, which comprises the step of screening a protein related to an arbitrary disease or biological function by measuring the amount of the group of the monitoring molecules by using the set of antibodies described in the abovementioned 31.
33. The method described in any one of the aforementioned 1 through 27, which verifies a validity of the target protein as a candidate for a target for drug discovery, a candidate for a protein drug, or a candidate for an antigenic protein for an antibody drug for the purpose of development of a drug for an arbitrary disease or a symptom, by measuring the amount of the group of monitoring molecules by using the set of antibodies described in the abovementioned 31.

### Best Mode for Carrying Out the Invention

In the specification, an arbitrary protein that is an object of a method of the present invention is referred to as a "Target Protein." In order to apply the method of the present invention, the target protein needs to have information on amino acid sequences. Information on biological functions is not always necessary in order to apply the method of the present invention. However, it is convenient that biological functions and a protein family to which it belongs are predicted beforehand based on the sequence homology, characteristics on a sequence related to the function, and an existence of a motif sequence, by searching a protein database with known functions. Recently, novel proteins are isolated and purified without elucidation of their functions, and numbers of proteins have been increasing whose three-dimensional structures are revealed by crystal analysis or nmr analysis. Information such as biochemical functions and existence of a site to which a small molecule ligand binds, which can be predicted from the three-dimensional structure, may be helpful in predicting a relation between a biomolecule network and biological response data. When a sample of the target protein is available, a specific antibody can be prepared and used as a probe means. When a crystal structure (a three-dimensional structure predicted by nmr) of an analogous protein is not available which is appropriate for use as a template for modeling the target protein, it is possible to use a specific ligand obtained by an experimental random screening as the probe means.

The meanings and definitions of the terms in the specification are as follows:

The term "specific ligand" or "ligand" means a small molecule or a small-molecular compound which binds to a target biomolecule (mainly to a protein) with a certain strength.

The term "biomolecule network" information means a network indicating a functional, biosynthetic, or metabolic linkage among biomolecules. In principle, a linkage is assumed to exist between biomolecules that bind directly or interact to each other. For example, linkages wherein, for example, molecule A becomes a substrate for molecule B or wherein a signal is transmitted by binding of molecule A to molecule C are referred to as functional linkages, and linkages wherein, for example, molecule C is generated from molecule A by an enzyme molecule B are referred to as biosynthetic linkages. Typical examples of molecule network-type database focusing on the molecular linkage include, for metabolic pathways, KEGG(Kanehisa et al., Kyoto University), Biochemical Pathways (Boehringer Mannheim), WIT (Russian Academy of Sciences), Biofrontier (Kureha Chemical Industry) ,Protein Pathway (AxCell), bioSCOUT (LION), EcoCyc (DoubleTwist), UM-BBD (Minnesota Univ.).

PATHWAY database of KEGG contains metabolic pathways of general small-molecular compounds participating in substance metabolism and energy metabolism and proteins in signal transductions. Those for signal transductions, CSNDB (National Institute of Health Sciences, Japan) , SPAD (Kuhara et al. Kyushu Univ.) , Gene Net (Institute of Cytology & Genetics Novosibirsk, Russia), GeNet ( Maria G. Samsonova) are available. As database for protein-protein interaction, DIP (UCLA), PathCalling (CuraGen), ProNet (Myriad) are available. The inventors of the present invention filed a patent application directed to a method for generating molecule function network including information on an event caused by a specific biomolecule in an organism, an organ, a tissue, and a cell, in addition to information on linkages among biomolecules ("Method for Generating Molecule Function Network", the specification of Japanese Patent Application No. 2000-276699). Molecule function network described in the aforementioned specification is included in the biomolecule network of the present description.

The term "virtual screening" means a selection of a molecule with a high probability to become a ligand theoretically on computer from a huge amount of group of compounds in a compound database, instead of an experimental screening (HTS) by a binding to an arbitrary protein or an inhibition of a reaction and the like by using an enormous compound library. A method based on structural characteristics of a compound or a method based on the presence or absence of a partial structure is available, as well as a method which predicts a possibility of fitting to a ligand binding site in a target biological macromolecule.

The term "biomolecule" means a molecule existing in an organism, including an endobiotic organism, such as an organelle, a cell, a tissue, an organ, a living individual, or an aggregate. The term means an organic molecule of any structure such as a nucleic acid, a protein, a lipid, a carbohydrate, an ordinary low molecular weight compound and the like, and a aggregate thereof, and may contain a metal ion, water, or a proton.

The term "biosynthesis" includes all reactions or processes where a biomolecule is generated in an organism, and may be used as a concept including a metabolism and chemical conversion of a small molecule, as well as a whole process of a protein synthesis.

The term "bioevent" is a concept including all phenomena, responses, reactions, and symptoms that are observed endogenously and exogenously in an organism. Examples of specific examples include transcription, migration of cells, adhesion of cells, cell division, neural circuit excitation, vasoconstriction, blood pressure increase, decrease in blood glucose level, fever, convulsion, infection by a parasite such as a living organism of different species and viruses.

The term "biological response" includes all biological phenomena caused in an organelle, a cell, a tissue, an organ, an animal body and the like by an application of the probe means to an arbitrary biomolecule, and should be interpreted in the broadest sense. Examples include, for example, an improvement of a disease index or a morbid index, a fluctuation of the amount of a monitoring molecule, an influence on a protein expression and a gene expression.

The term "biochemical function of a protein" means a function of a protein at a molecular level such as an enzyme reaction and a mode of transduction in signal transduction. Proteins are classified by the mode of enzyme reaction or the mode of signal transduction. For example, an enzyme which phosphorylates a protein is classified as a protein kinase, an enzyme which hydrolyzes a protein is classified as a protein hydrolase, a receptor which has 7 alpha-helixes penetrating a membrane to transmit an extracellular signal into a cell is classified as a 7-transmembrane receptor, and a protein which controls a transcription by a binding of sex hormones and others in a nucleus is classified as a nuclear receptor.

The term "biological function of a protein" is a function observed at a cell level or at a higher level. For example, both an acetylcholine receptor and an oxytocin receptor are proteins of 7-transmenbrane receptors, however, an acetylchline receptor is involved in a biological function of neurotransmission, whilst an oxytocin receptor is involved in a biological function of an oxytocic action. Angiotensin converting enzyme is a hydrolase of a protein (or a peptide), however, the enzyme converts angiotensin I to angiotensin II and is involved in a biological function of regulating blood pressure.

The term "small molecule" or "low molecular weight compound" means an organic compound with a molecular weight of preferably 200 to 1000.

The term "antisense molecule" means an oligonucleotide or oligoribonucleotide with about 10 to 30 residues having a complementary sequence to an arbitrary gene or a mRNA sequence, which is used to suppress a transcription and a protein expression and others by binding specifically to the gene or mRNA.

The term "probe means" is a means of inhibiting or modulating a function of a target protein such as a ligand which binds specifically to the target protein, an antisense molecule to a mRNA coding the protein, a vector to knockout a gene of the target protein, a vector containing a gene sequence coding the target protein, double stranded RNA to cause an RNA interference, a ribozyme designed to break a mRNA coding the target protein, and a specific antibody to the target protein.

The term "application of a probe means" is to inhibit or modulate a function of a target molecule by applying the probe means to an organism. For example, it includes applying a specific ligand, an antisense molecule or a monoclonal antibody to a cell, generating a mouse in which a target molecule is knocked-out, generating a mouse in which a target molecule is over-expressed, and injecting a specific antibody to a target molecule into a cell.

The term "biological response data" includes a fluctuation of the expressing amount or existing amount of a biomolecule and a physical parameter. As examples of the former, a fluctuation of protein expression, modification, mRNA expression, and an amount of a diagnostic marker molecule are pointed out, and as examples of the latter, blood pressure, body temperature, and urine volume and others are pointed out.

The term "monitoring molecules" is a group of molecules comprising one or more, preferably two or more, molecules selected or particularly designated for observing biological responses. It is desirable that a monitoring molecule is a biomolecule contained in the known biomolecule network information, however, it is not always necessary. In that case, it is desirable that a linkage with a molecule included in the known biomolecule network information has been elucidated by a certain technique such as the yeast two-hybrid method. It is possible to use, for each target protein, different monitoring molecules such as a group of proteins or a group of mRNA whose expression increases or decreases largely by an application of the probe means. Alternatively, it is also possible to use a certain group of molecules designated in advance with a certain intension for many target proteins as the monitoring molecules and measure a biological response. A monitoring molecule is not necessarily limited to a protein or mRNA, and it can be selected from all types of other small molecules, for example, from diagnostic marker molecules related to a disease, such as glucose and creatine.

The term "bioevent" is a concept that includes all phenomena, responses, reactions and symptoms occurring endogenously or exogenously in an organism. As actual examples, transcription, cell migration, cell adhesion, cell division, neural circuit excitation, inflammation, vasoconstriction, blood pressure increase, decrease in blood glucose level and others are pointed out.

The term "behavior of a monitoring molecule" or "behavior of a biomolecule" means a change in an amount, an expression level, a modification condition, localization, binding state of a cofactor and others of a monitoring molecule or a biomolecule.

The term "biomaterial" means a sample of any level contained in an organism such as an organelle, a cell, a tissue, and an organ.

In the following, one of the embodiments of the present invention, a method for the purpose of a validation of a target for drug discovery, is explained with a flow chart in detail. However, the method of the present invention is not limited to the following method and its detail. This method can be carried out not only for validating a target protein for which a specifically-binding drug molecule is to be developed, but also for validating a target protein wherein the protein itself is used as a drug or for which an antibody drug is to be developed using the target protein as an antigen.

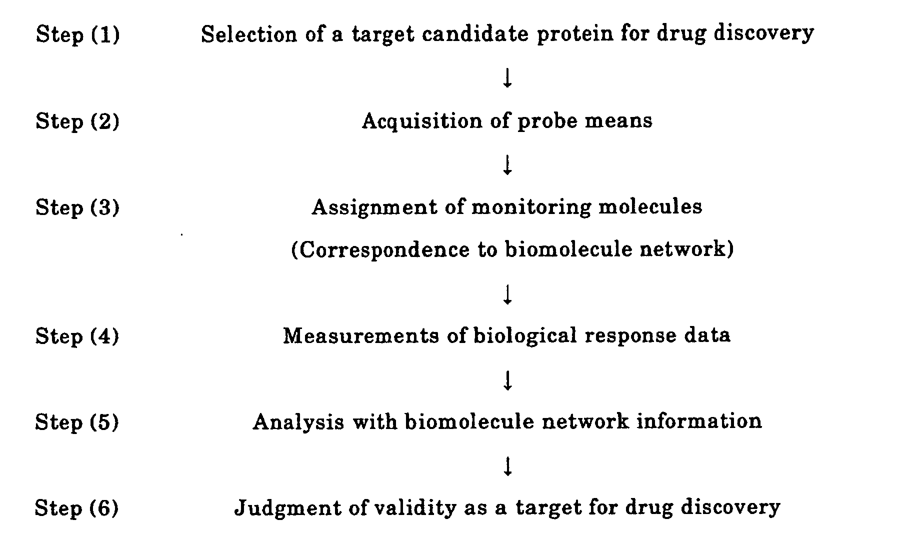

### Step (1): Selection of a target candidate protein for drug discovery

When the present invention is used for judgment of validity of an arbitrary protein as a target for drug discovery, it is possible to select a target candidate protein for drug discovery by the following method beforehand. As a group of target candidate proteins for drug discovery, it is not necessary that a biological function of each protein is known, which is the characteristic of the method of the present invention. There need be no relationship among the proteins to be treated, and one may use a group of independent proteins that are collected from literatures by drug discovery researchers, or one may identify and select a group of proteins with remarkable increase or decrease of expression between patients of a specific disease (symptom) and normal people from two-dimensional electrophoresis map of the expressing protein. By using the biomolecule network information indicating a functional or biosynthetical relation between biomolecules, one can predict a group of proteins directly or indirectly related to proteins with remarkable increase or decrease of expression upon a specific disease and select them as a group of target candidates for drug discovery, rather than selecting the increasing or decreasing proteins themselves. Furthermore, by using the biomolecule network information including information of bioevents such as vasoconstriction that are caused by specific biomolecules, one can select a group of proteins on the biomolecule network, that are connected to a bioevent related to a morbid state or a symptom of the target disease, as target candidates for drug discovery. However, it is needless to say that the present invention is applicable to target candidates for drug discovery selected by any other methods, which are not included in known biomolecule network information.

An example of selecting a group of proteins of target candidate for drug discovery by using the molecule network is shown below.

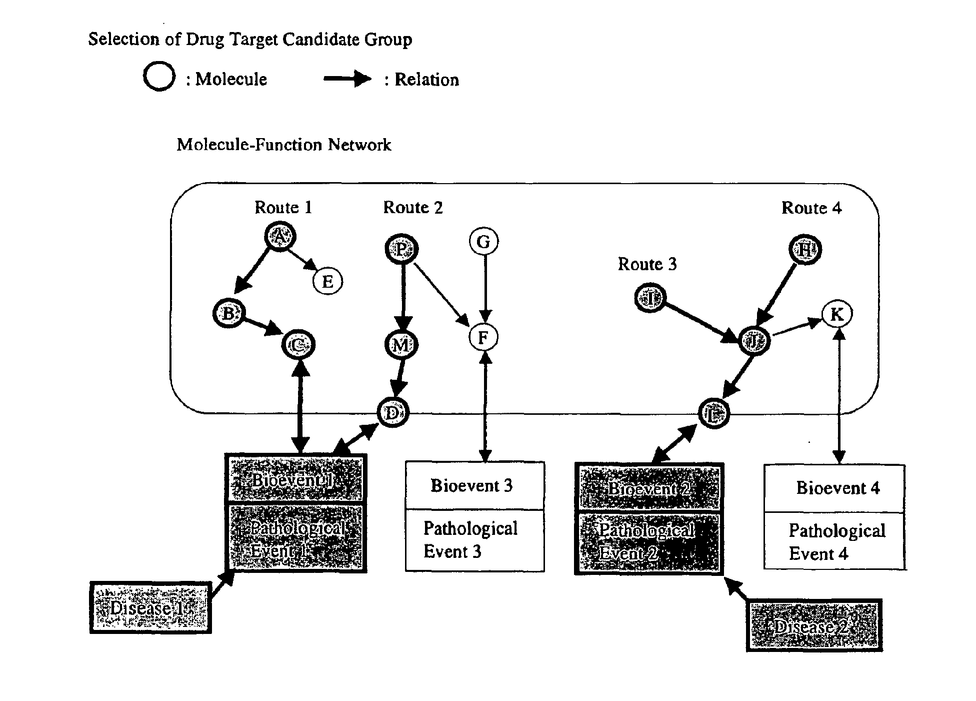

Assume proteins existing on the molecule network related to a disease as target candidates for drug discovery.
For "Disease 1", from Route 1 and Route 2,
there are 6 target candidates, "A, B, C, D, M, P".
For "Disease 2", from Route 3 and Route 4,
there are 4 target candidates, "H, I, J, L".

### Step (2): Acquisition of probe means

As typical examples of probe means, there is a method of using a specific ligand, a method of using an antisense molecule, and a method of using a monoclonal antibody. A specific ligand binds specifically to the ligand binding pocket of the target protein and other sites and controls its function. While any method may be used to create a specific ligand, there is a method of finding it experimentally by a random screening and a method of using a virtual screening. For finding a ligand experimentally, steric structure information of the target protein is not necessary, however, it is necessary to prepare protein sample and a huge numbers of compound library and carry out a large-scale random screening. For a protein whose function is unknown and whose sequence information is only available, it is often necessary to produce and purify the target protein from the gene and to employ a method of directly measuring the binding to the target protein because a screening method of measuring the inhibition of enzyme activity or the competition with a known ligand cannot be used.

As a method of creating a specific ligand by a virtual screening, any method can be used as long as it can find ligands with various structures as many as possible. From a viewpoint of a small molecule which binds stably to a ligand binding pocket of a target protein, it is desirable to use a virtual screening method based on an automatic docking, which is able to search novel ligands with wide variety of structures without preconception. More preferably, it is possible to use "Method of Retrieving Novel Ligand Compounds from Three-Dimensional Structure Database" (Japanese Patent Application No.8-514452) which is a method of searching a compound database using the automatic docking method whose name of the invention is "Method of Searching the Structure of Stable Biopolymer-Ligand Molecule Composite" (Japanese Patent No.2621842). This method makes it possible to predict, for each compound, a stable complex structure with a target protein considering all degrees of freedom arising from binding modes and ligand conformations, and to search compounds with stability and characteristics of their structure as search criteria. It has been proved that compounds selected by this method show the desired activity highly frequently in many protein systems.

As a target for virtual screening, any compound database may be used. When a compound database with information on two-dimensional structures is available, it is possible to convert it automatically to a three-dimensional database having necessary information such as the three-dimensional structures by using a software for three-dimensional structure conversion, and to use it for the aforementioned virtual screening. If a commercial compound database is used as a target, it is convenient that promising compounds (hits) selected by the search can be obtained and provided for the following experiment without synthesizing the compounds. Besides commercial compounds or existing compounds, one may search a database of compounds having appropriate chemical structures for exploring a biological response as a ligand used as a probe means. In this case, the hit compound needs to be synthesized.

When a compound selected by a virtual screening is obtained, it may be provided for the following experiment directly. However, depending on the reliability of the method of the virtual screening, or on the other conditions such as the shape of the ligand binding pocket, it is desirable, if possible, to confirm a binding activity to the target protein experimentally and then proceed to the next step. Binding activity to the target protein of the compound selected and obtained by the virtual screening can be confirmed by a method based on a surface plasmon resonance and others, if a protein sample is available.

If a specific ligand is to be created by a virtual screening, information on the steric structure of the target protein is necessary. When a crystal structure or an nmr structure (3 dimensional coordinates of each atom) of the target protein itself is available from the Protein Data Bank and others, it can be used directly for the virtual screening. Even though the crystal structure of the protein has not been analyzed or its three-dimensional coordinates are not available, when information on the crystal structure of an analogous protein is available, one can use a structure modeled on a computer by techniques such as substituting side chains of the analogous protein using its crystal structure as a template, as well as the crystal structure. The higher the sequence homology, the higher the possibility of similarity of the steric structures, and the easier the modeling. However, even if the homology of the entire sequence is not so high, as long as a motif sequence or a characteristics in the sequence related to a biological function exist, it is known that they belong to the same protein family and have similar steric structures. Thus, if a crystal structure of the protein belonging to such a family is available, the modeling is possible. Furthermore, when information on a steric structure of a protein with a high homology of sequence to the protein is not available, one can search a protein structure that can be a template by considering properties other than the sequence homology, and can model the three-dimensional structure. As a typical method, 3D-1D method by D. Eisenberg and others is pointed out.

Even though a ligand is created by any methods such as an experimental method or a virtual screening method, it is desirable to confirm its properties such as water solubility and fat solubility and absence of significant cytotoxicity, before providing it to a measurement experiment of a biological response. When a ligand is weak in binding to the target with the found structure as it is, it is desirable to improve binding activity and properties such as solubility by modifying the structure as necessary. More preferably, it is recommended that multiple specific ligands with different structure characteristics are used for the same experiment and compare the biological responses, rather than using a single specific ligand. It is desirable to confirm that an influence on expression of proteins other than the target protein and an amount of the monitoring molecule, or a correlation with the biomolecule network predicted by those data, are common for the ligands.

As a probe means, an antisense molecule can be used instead of a specific ligand. Generally, an antisense molecule is an oligonucleotide of about 10 to 30 residues, binds selectively to mRNA that has a complementary base sequence, and is used to inhibit a translation of genetic information and a protein expression. Whereas a specific ligand inhibits or activates the function of the target protein directly without giving any influence on the expression or the amount of the protein, the antisense molecule has an effect of inhibiting a synthesis of the protein directly. Consequently, by synthesizing and administering the antisense molecule having a complementary base sequence to mRNA corresponding to the amino acid sequence of the target protein, it is possible to explore a role of the protein in an organism from influences on other biomolecules as a result of inhibiting the synthesis of the protein.

Therefore, biological responses are different when a specific ligand, an antisense molecule, or a monoclonal antibody are given to an organism, and it is useful to give two or three kinds of such probe means for predicting a function and a disease related to the protein. In case of the target protein for which a small-molecular specific ligand cannot be created, an antisense molecule can be used as a probe means. Moreover, an antisense molecule can be synthesized easily as long as mRNA sequence coding the target protein is available, and there is an advantage that steric structure information is not necessary. However, when the target protein is an already synthesized protein, a protein with a slow turnaround, or a protein developing an important physiological function upon conversion such as hydrolysis from a precursor protein already synthesized, it is possible that quite different biological responses are observed compared to the use of a specific ligand. Especially, by measuring and comparing biological responses to an antisense molecule and a specific ligand with two or more elapsed times after applying the probe means, it is highly possible that a clue about the property of the target protein is obtained. Therefore, as a preferred embodiment of the method of the present invention, a method of preparing two or more specific ligands as the probe means and further creating an antisense molecule or a monoclonal antibody can be pointed out.

### Step (3): Assignment of monitoring molecules

As data of a biological response when an application of the probe means is given to a cell, a tissue, an organ, an animal body and others, any data are acceptable as long as a difference with or without the application of the probe means can be measured and compared. As examples of such data, 1) influence on blood pressure, body temperature, pulse, shape of a cell, growth rate and others, and 2) influence on expression or existing amount of biomolecule can be considered. Generally, since type 1) is difficult in interpreting the data, type 2) is preferable wherein the influence on expression or amount of biomolecules for which a measurement is easy and a lot of data can be obtained at one time is taken as a biological response data. A molecule for the measurement of a biological response of type 2) to the application of the probe means is called a "monitoring molecule."

The simplest method of choosing a monitoring molecule is to select a protein or mRNA whose expression or modification changes remarkably upon application of the probe means. It is a routine technique to determine with mass spectroscopy amino acid sequences of a group of proteins whose expression levels increase or decrease remarkably between patients and normal people, or upon administration of a drug. The problem is, that even though there are many proteins whose expression levels vary remarkably, they are not effective means for predicting biological functions because relationships among them are unknown. In order to know functions of a protein whose biological functions are unknown, there is no choice but to correlate biological functions and diseases with known proteins. Therefore, it is desirable to select as monitoring molecules, proteins or mRNAs whose expression or modification change remarkably upon the application of the probe means, and that are included in the biomolecule network indicating functional or biological linkages between biomolecules. With the biomolecule network information, one can correlate directly or indirectly among many proteins whose expression varies remarkably upon the application of the probe means and which are apparently unrelated, and one can locate the target protein among biomolecules with known biological functions. In this method, a combination of proteins treated as monitoring molecules varies for each target protein, and a set of monitoring molecules is determined after observing changes in protein expressions upon the application of the probe means.

In a preferred embodiment of this method, proteins on the protein expression map are corresponded to molecules on the known biomolecule network as much as possible. By doing this, correspondences with molecules on the known biomolecule network are prepared to any combination of monitoring molecules, and it becomes easy to analyze a relation between a target protein and biomolecules with known functions for any target protein. Alternatively, mRNAs with large fluctuations on a mRNA expression map upon the application of the probe means may be used as monitoring molecules, not depending on the protein expression map. However, it must be kept in mind that the scope of the method of selecting monitoring molecules based on protein expression or mRNA expression is limited to proteins.

Monitoring molecules can be a group of arbitrary biomolecules including small molecules that are intentionally selected, not based on the protein expression map and the mRNA expression map. It is desirable that these molecules are included in the biomolecule network information, and furthermore, it is desirable that they are corresponded to molecules on the known biomolecule network.

It is desirable that one or more, preferably several dozens of, monitoring molecules are assigned to one target protein, however, any combination of molecules is acceptable. For example, it is possible to choose one or more groups of molecules properly from a group of molecules that are known to be indices of a specific disease or to increase or decrease in a patient of a certain disease, from a group of proteins whose expressions increase or decrease remarkably between patients of a specific disease and normal people, from a group of molecules that exist characteristically in a specific organ or a specific site of a specific tissue, from a group of biomolecules that bind to receptors, or from a group of molecules included in a certain molecular pathway in the biomolecule network. For instance, examples of selecting monitoring molecules based on correlations with a specific disease or expressions in a specific organ are as follows. 1) If a measurement system for quantitatively measuring and/or detecting a set of all proteins and small molecules known to be correlated with immunity is established, it is possible to find or screen a novel protein related to an immune system, which leads to a finding of a novel target for drug discovery of the field. 2) If a measurement system for quantitatively measuring and/or detecting a set of all proteins and small molecules known to be correlated with the central nervous system is established, it is possible to find or screen a protein which is a target for drug discovery, for example, for Parkinson's disease and dementia.

### Step (4): Measurement of biological response data

A biological response of a cultured cell, a cultured tissue, a cultured organ, an animal body, or others to the application of the probe means may be measured by a behavior of the monitoring molecule in the administered cell, tissue, organ, animal body or others, for example, by a change of the expression level and/or the amount. It is also possible to measure the biological response to the application of the probe means administered to an animal body, for instance, by a behavior (for example, a change of the expression level and/or the amount) in a specific biomaterial (for example, an organ, a tissue, a cell and others).

Detection and/or quantitative measurement of the monitoring molecule may be carried out by any method, however, it is desirable that the method has high sensitivity and high accuracy in order to detect and/or quantify the molecule existing in a small amount. If an antibody is prepared using each monitoring molecule as an antigen, the amount of the monitoring molecule can be determined easily with high sensitivity by a technique such as ELISA and Western blotting. Measurement of many monitoring molecules at the same time is also possible. Consequently, as a preferred embodiment of the present invention, it is desirable that a set of antibodies is prepared to a set of monitoring molecules (a group of monitoring molecules) selected with a specific intension, and they are selected properly according to the purpose. For example, if one use a set of monitoring molecules combined as to include molecules involved in various physiological functions as much as possible, one can predict a correlation between the target protein and a physiological function by measuring which molecule is strongly affected in its amount. Furthermore, if one uses a set of antibodies to a group of monitoring molecules comprising molecules related to a specific disease, one can examine whether the target protein is related to the specific disease with the effects given to molecules in the set by the target protein. Moreover, if one measures biological response data using one or more sets of antibodies similarly prepared according to a disease of interest, one can discover a novel target for drug discovery. Measurement of the behavior of the monitoring molecules may be carried out using any means. For example, one may use a DNA chip on which corresponding DNA probes are immobilized when fluctuations of mRNA levels are to be measured, and when fluctuations of the protein levels are to be measured, one may use a method of separating the extracted proteins by two-dimensional electrophoresis or liquid chromatography, for example, and measuring the amount of the proteins. Furthermore, one may consider a method of using electronic spectrum and others and identifying monitoring molecules by comparing with a spectrum of a standard sample of each molecule measured in advance.

One may measure expressing amount or existing amount of the monitoring molecule with and without the application of the probe means, and take its fluctuation (difference) as a biological response data. Biological response data may be obtained by measuring in a time course, at different elapsed times after the application of the probe means, for example, 5 min., 20 min., 1 hour and 4 hours. Furthermore, it is desirable to apply the probe means at 2 or more doses or concentrations, measure biological response data, and to be used for analysis. Moreover, for a protein for which one can create its specific small-molecular ligand, one may measure and analyze biological response data, as a preferred embodiment of the present invention, by applying both the antisense molecule and the specific ligand as the probe means, or by applying two or more specific ligands having different structural characteristics.

### Step (5): Analysis using biomolecule network information

Biological response data described for two or more monitoring molecules on the known biomolecule network are the strength of the response and the time of appearance of the fluctuation. The stronger the biological response and the shorter the time of appearance of the fluctuation, relation of the target protein with the molecule on the known biomolecule network can be regarded closer. In other words, it is possible to predict that the target protein is close to which molecule of which molecular pathway in variously branched linkage of biomolecules (molecular pathway) in the known biomolecule network.

Furthermore, considering the information whether the closest monitoring molecule is a receptor, a ligand, an enzyme, or a substrate, and considering a biochemical function of the target protein predicted from a motif search, steric structures or others, it is possible to predict relation between the target protein and molecules in the known biomolecule network. From the closest molecule or molecular pathway, it is possible to predict a biological function to which the target protein is related. If the biomolecule network information having bioevent information is available, it is possible to predict a relation with a disease based on the bioevent information to which the closest molecular pathway or molecule is connected. If an evaluation score is calculated based on the number of intervening biomolecules and the reliability of information on the direct interactions between biomolecules, it is possible to rank cases wherein relations with two or more molecular pathways or molecules are predicted.

As existing network-type databases of biomolecules, KEGG (Kyoto University, Kanehisa et al.) and GeneNet (Institute of Cytology & Genetics, F. A. Kolpakov) store molecules on the metabolic pathways or known molecules involved in the signal transduction. However, these existing databases do not store bioevent information above cellular level (for example, contraction of smooth muscle or hypoglycemic action) although they contain information on actions of each molecule at molecular level (for example, phosphorylation or binding to a receptor). Therefore, relation with a disease, a symptom, and a side effect cannot be suggested from these databases. As biomolecule network information for use in the method of the present invention, it is convenient to use those containing information on bioevents such as a biological response above cellular level caused by a biomolecule and information whether the bioevent rises or lowers by a specific molecule, with which one can easily understand biological response and disease to which a target protein is related directly or indirectly, as well as the information on linkages between biomolecules and the information on actions at biochemical level. As an example of such a network-type database of biomolecules, KeyMolnet (Title of Invention: "Method of Forming Molecular Function Network" Japanese Patent Application No.2000-276699) is available.

When another protein that interacts directly with the target protein is known by the two-hybrid method and other methods, it is possible to consolidate the prediction for the target protein about the position in the biomolecule network and the relation to a function or a disease, by obtaining a probe means for the said another protein and measuring and comparing biological response data, similarly with the target protein, and it is possible to predict a relation with a function and a disease for two or more proteins with unknown biological functions.

When it is suggested, as a result of the analysis, that the linkage between the target protein and the biomolecule network, which is a linkage between biomolecules with known biological functions, is indirect and other one or more biomolecules might intervene, it is possible to clarify the relation to the known biomolecule network by carrying out the aforementioned process for the said other molecules one after another. For example, by carrying out the aforementioned process for protein B whose expression fluctuates remarkably by a specific ligand or an antisense molecule for protein A, and by carrying out a similar process for protein C, it is found that protein A has a direct relation with protein B, protein B has a direct relation with protein C, and protein C coincides with one of the proteins in the known biomolecule network. By repeating the aforementioned process until it arrives at a molecule in the known biomolecule network, it is possible to obtain information on a linkage of the target protein to one of the molecules in the known biomolecule network, and also to identify intervening biomolecules. Among proteins on a two-dimensional electrophoresis map of protein expression, there are many that cannot be added to the biomolecule network information because their functions are unknown although their amino acid sequences can be determined. Consequently, even if the fluctuation of the protein expression is remarkable, it is difficult to utilize those information. However, one of the characteristics of the method of the present invention is that one can add those to the biomolecule network information by repeating the aforementioned process and can expand the network information.

### Step (6): Judgment of validity as a target for drug discovery

This step can be carried out additionally as necessary. Judgment of validity of the target protein as a target for drug discovery is straightforward, when it is judged easily with an improvement of the clinical index, or the amount of monitoring molecule and others in an animal. However, in cases where it is not applicable or evaluation is done by the protein expression or corresponding mRNA expression, it is desirable to judge by using the result of step 5 which uses information on the molecule network indicating relations between the biomolecules related to the said protein functionally or biosynthetically. By this method, it is possible to analyze whether the expression of the protein related in the molecule network changes in a favorable direction by the application of the probe means. A kind of biomolecule network information employed is not particularly limited. However, it is desirable to use biomolecule network information containing a biological response above cellular level as a bioevent information, such as the biomolecule network information in the "Method of Forming Molecular Function Network" (Japanese Patent Application No.2000-276699). Molecule linkage from a target candidate protein for drug discovery to a bioevent or a biomolecule that is a direct cause of the target disease or symptom is made clear, and a linkage with other bioevents can be predicted.

One of the necessary conditions as a drug target is that the risk of side effect is low. Among side effects, there are those caused as a consequence of the selection of the target for drug discovery, and those caused depending on the structure of a compound (ligand). In the latter case where the side effect is caused by binding to an irrelevant biomolecule in the biomolecule network, it is possible to avoid it by further structure modification or a search for a ligand with completely different structure. However, it is difficult to avoid the side effect in the former case, and thus it is not appropriate to use the protein as the target for drug discovery. The former case can be predicted to some extent from the biomolecule network information, but it is useful to check the balances with the main actions experimentally with a specific ligand and/or an antisense molecule. By using the biomolecule network information with bioevents, it is possible to recognize the existence of other biomolecule network to which a bioevent resulting in a side effect is related.

Furthermore, as there are cases in which clearance by metabolism is very rapid depending on the ligand structure, decomposition by metabolic enzymes and others may be checked as necessary. When there are experimental results in a body, an organ, a tissue, and a cell for an animal wherein a gene of the protein is knocked out, it is desirable to compare its effect with the effect by administration of a specific ligand and/or an antisense molecule. Moreover, when the activity strengths or the properties such as a solubility and others of a group of specific ligands created by HTS or virtual screening are not sufficient, one may search an analog compound of the group of ligands from the commercial compounds or to use a compound which is synthesized and improved.

The features of the method of the present invention can be summarized as follows.

By relating the target protein for which information other than sequence is not available to the biomolecule network information which indicates functional or biosynthetical linkages between biomolecules with known biological functions, it is possible to predict biological functions and/or diseases related to the target protein.

As a means of the relating, effects on the amount of the group of monitoring molecules, which are preferably molecules in the known biomolecule network, are measured and analyzed as biological response data, using a specific ligand or an antisense molecule of the protein as the probe means.

As well as a group of proteins whose expression of protein or mRNA is significantly affected by the application of the probe means are used as monitoring molecules, a group of arbitrarily selected monitoring molecules can be used as monitoring molecules, and by including small molecules into a group of monitoring molecules, it is possible to detect and/or quantify proteins with low expression level.

By preparing a set of antibodies produced for two or more monitoring molecules, it is possible to measure many monitoring molecules with high sensitivity at the same time.

By predicting direct and indirect linkages of the target protein with the molecules in the known biomolecule network based on the analyses of the time course of the amount of the group of monitoring molecules and others, it is possible to predict biological functions and/or diseases with which the target protein is related.

By using a group of monitoring molecules selected with an intention as the monitoring molecules, it is possible to screen a protein related to a specific disease or a specific biological function from many groups of proteins with unknown functions.

By using the biomolecule network information containing bioevent information, it is possible to predict a relation with a disease from the linkage between the target protein and a molecular pathway in the network.

By carrying out the aforementioned analyses, it is possible to predict whether the target molecule is appropriate as a target for drug discovery, or what kind of side effect occurs when a drug is developed using the target molecule as a target for drug discovery.

As one of the embodiments of the method of the present invention, the following examples are pointed out.

### Example of using specific ligand

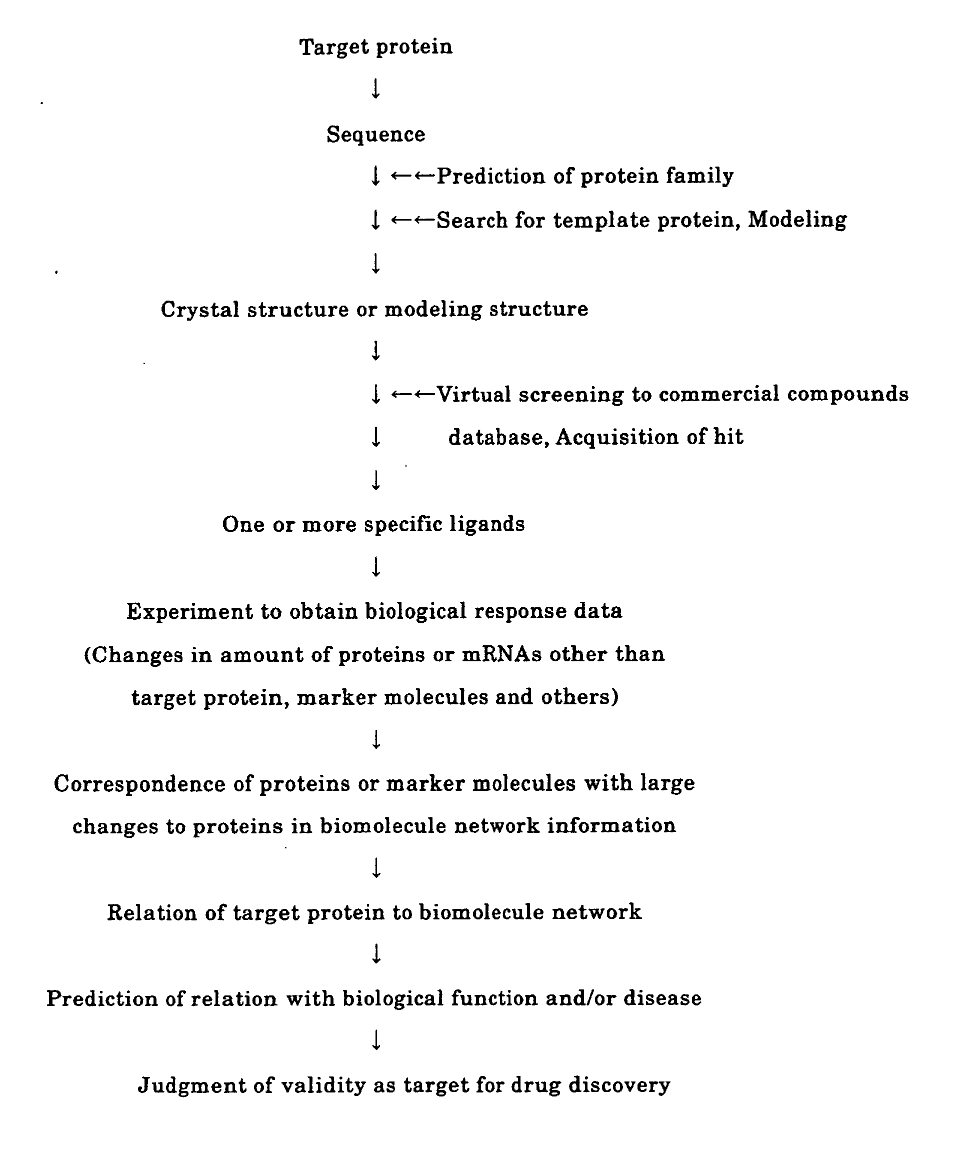

### Example of using antisense compound

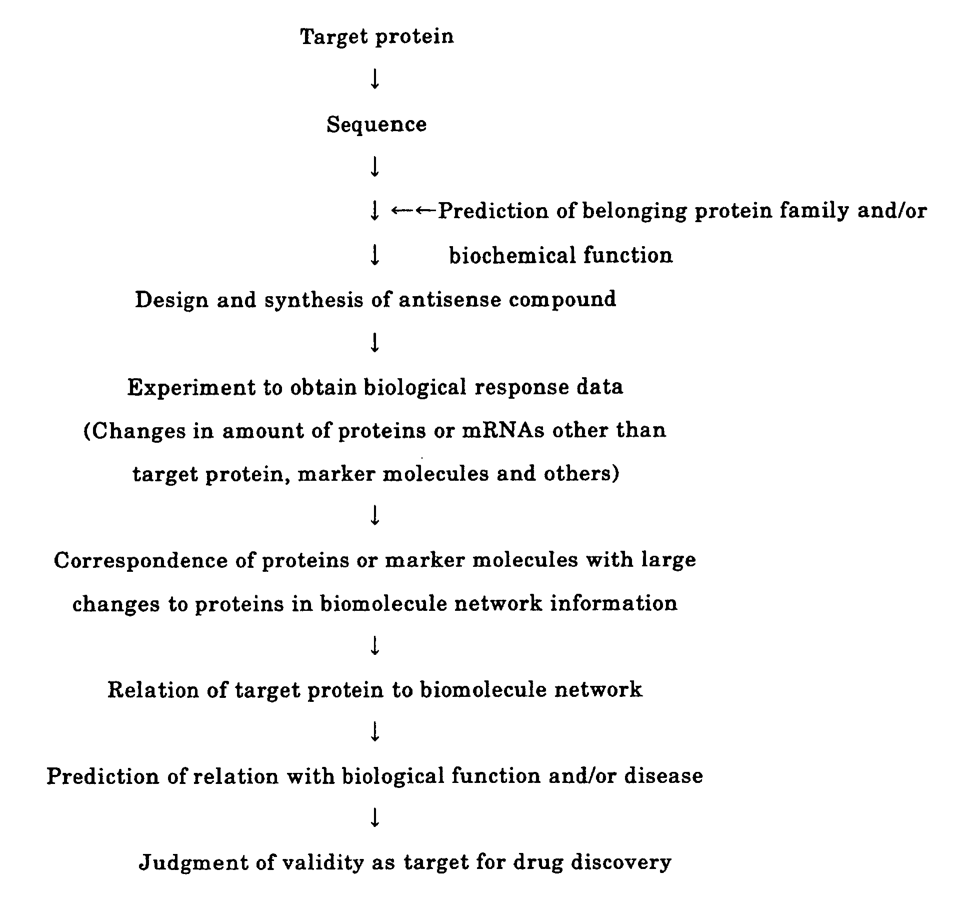

Biological function of the target protein predicted by the method of the present invention is not limited to one, rather, it is possible to predict to what kind of function or what kind of disease the target protein is related by carrying out the method of the present invention. Biochemical function predicted from the sequence information can also be utilized in the method of the present invention. Biological function of a biomolecule is determined by a relation with other biomolecules and it cannot be elucidated even if the molecule itself (only that molecule) is studied in details. On the other hand, the method of the present invention has characteristics that one can predict a function of the target protein easily by measuring and analyzing the effects to an organism itself or expression and amount of biomolecules other than the said protein as biological response, by using a specific ligand of the said protein or an antisense molecule to the gene of the said protein and others as the probe means. Furthermore, by using the biomolecule network information which indicates functional or biosynthetical relations between biomolecules with known biological functions, and by analyzing and predicting with what molecule on the network that the target protein has a close relation, it is possible to carry out a functional prediction more effectively.

Generally, an amount and a function of a biomolecule are controlled complicatedly by mechanisms such as a feedback control, and there are cases in which an effect to one molecule may eventually extend to many molecules. Therefore, by measuring biological response data at various elapsed times after the application of the probe means, it is possible to predict a molecule or a molecular pathway in the network having a close relation with the target protein, and based on the biological function or the disease with which the molecule or the molecular pathway is related, it is possible to predict roughly what biological functions and diseases the protein is related to.

By carrying out such analyses, it is possible to evaluate validity of a protein as a target for drug discovery, to evaluate a risk of side effect when a drug molecule is developed with the protein as a target for drug discovery, and to obtain information for selecting an appropriate target for drug discovery, before starting a full-scale drug discovery project such as HTS. Therefore, the present invention is also useful as a method of validating a target for drug discovery. When a specific ligand is used as the probe means, the method is more useful since one can obtain evaluation information on the target for drug discovery accompanied with information on drug lead.

### Industrial Applicability

By the method of the present invention, one can predict functions of a protein, to be more specific, biological functions and/or diseases to which the protein is related, and for example, one can easily confirm whether the protein is appropriate or not as a target for drug discovery.

## Claims

1. A method for predicting a biological function and/or a disease to which a target protein is related, which comprises the steps of applying a probe means, which is based on an arbitrary target protein, to a biomaterial or an animal, and by measuring, and analyzing an effect on a behavior of one or more monitoring molecules each with a known or unknown biological function.

2. The method according to claim 1, wherein the target protein is that with an unknown biological function.

3. The method according to claim 1 or 2, wherein the behavior of the monitoring molecule is a quantitative or a temporal fluctuation in an amount of expression and/or an amount of presence, and the probe means is applied to a cell, a tissue, an organ, or an animal.

4. A method of predicting a biological function and/or a disease to which a target protein is related, which comprises the steps of using a probe means, which is based on an arbitrary target protein, and measuring an effect on a behavior of one or more monitoring molecules each with a known biological function as a biological response datum, and then analyzing a direct or an indirect relation of the target protein with a molecule included in a known biomolecule network information.

5. The method according to claim 4, wherein the target protein is that with an unknown biological function.

6. The method according to claim 4 or claim 5, wherein the behavior of the monitoring molecule is a fluctuation in an amount of expression and/or an amount of presence.

7. The method according to any one of claims 1 through 6, wherein the probe means is a specific ligand and/or an antisense molecule.

8. The method according to any one of claims 1 through 7, wherein the monitoring molecule is a molecule included in a known biomolecule network information, and wherein said molecule is a protein or an mRNA each with a large degree of fluctuation in an amount of expression by application of the probe means and/or a group of specifically designated monitoring molecules,.

9. The method according to claim 8, wherein the group of monitoring molecules is a group of molecules selected so as to have relations with variety of biological functions as wide as possible.

10. The method according to claim 8 or claim 9, wherein the group of monitoring molecules is selected from a group comprising a group of molecules which relate to a specific disease and/or a biological function and a group of molecules which express and/or function in a specific organ or a specific tissue.

11. The method according to any one of claims 3 through 10, wherein the biomolecule network information is that indicates a linkage of each interaction in a function and/or a biosynthesis between biomolecules each with a known biological function.

12. The method according to any one of claims 3 through 10, wherein the biomolecule network information is a molecule network information including information on a relation between a biomolecule and a bioevent, in addition to the information indicating the linkage of the interaction in the function and/or the biosynthesis between the biomolecules each with the known biological function.

13. The method according to any one of claims 7 through 12, wherein the specific ligand to the target protein is created by a virtual screening.

14. The method according to claim 13, wherein the virtual screening includes a search process of a compound database based on an automatic docking considering binding modes and all degrees of freedom of ligand conformations.

15. The method according to claim 13, wherein the virtual screening is a search method of a compound database based on the automatic docking method ADAM.

16. The method according to claims 7 through 15, which comprises the step of administering two or more specific ligands to a single target protein or a combination of a specific ligand and an antisense molecule at two or more doses or concentrations, and measuring and analyzing a fluctuation in an amount of expression or an amount of presence of the monitoring molecule as a biological response.

17. A method for predicting a relation of a target protein with a biological function or a disease, which comprises the steps of searching each different protein with an unknown biological function, whose expression fluctuates remarkably by applying a probe means which is generated based on information on the target protein, and searching one or more proteins whose expression fluctuate remarkably by applying a probe means which is created for the different protein above obtained, and further repeating the aforementioned steps until one of the aforementioned proteins whose expression fluctuate remarkably become identical to one of molecules included in a known biomolecule network information to analyze a relation between the target protein and the molecule on the biomolecule network.

18. A method for predicting a relation of two or more proteins including a target protein with a biological function or a disease, which comprises the step of using each probe means created for the target protein and created for a different protein with an unknown biological function, wherein said different protein is known to interact directly with said target protein, to analyze an influence of an application of the probe means on a behavior of a monitoring molecule, and by analyzing a relation of said two or more proteins including the target protein with unknown biological functions with the molecule included in the known biomolecule network information.

19. The method according to claim 18, wherein the behavior is a quantitative and/or a temporal fluctuation of an amount of expression and/or an amount of presence.

20. The method according to any one of claims 1 through 19, wherein a validity of the target protein as a target for drug discovery, a validity of the target protein as a drug, or a validity of use of an antibody against the target protein as a drug is verified by measuring and analyzing the fluctuation of the monitoring molecule caused by the application of the probe means based on the target protein.

21. The method according to any one of claims 1 through 20, wherein a protein which can be a target for drug discovery, a protein which can be a protein drug, or a protein which can be an antigen for an antibody drug is selected based on the biomolecule network information for a purpose of development of a therapeutic drug for an arbitrary disease.

22. A method of selecting an appropriate candidate for an arbitrary disease by comparing two or more candidates for the target for drug discovery, candidates for a protein drug, or candidates for an antigenic protein for an antibody drug extracted based on the known biomolecule network information by applying any one of the methods according to claims 1 through 21.

23. The method according to any one of claims 1 through 22, wherein a side effect when a drug is developed by using the target protein as a target for drug discovery, a side effect when the target protein itself is developed as a drug, or a side effect when an antibody against the target protein is used as a drug is predicted by measuring and analyzing the fluctuation of the behavior of the monitoring molecule induced by the application of the probe means based on the target protein.

24. The method according to any one of claims 1 through 23, wherein the side effect is predicted for the protein which can be the target for drug discovery, for the protein which can be the protein drug, and for the protein which can be the antigen for an antibody drug for the purpose of development of a therapeutic drug for an arbitrary disease or a symptom based on the biomolecule network information.

25. An antibody set which can simultaneously quantify and/or detect the group of monitoring molecules according to any one of claims 1 through 21.

26. The method according to any one of claims 1 through 25, which includes the step of screening a protein relating to an arbitrary disease or a biological function by quantifying the group of the monitoring molecules by using the antibody set according to claim 22.

27. The method according to any one of claims 1 through 26, wherein the validity of using the target protein as a candidate for a target for drug discovery, as a candidate for a protein drug, or as a candidate for an antigenic protein for an antibody drug is verified for a purpose of development of a therapeutic drug for an arbitrary disease or a symptom by quantifying the group of the monitoring molecules by using the antibody set according to claim 22.
